# EUROPEAN PATENT APPLICATION

(11) **EP 4 414 141 A1**
(43) Date of publication of application: **14.08.2024**
(21) Application number: 22923360.6
(22) Date of filing: 31.10.2022
(51) Int. Cl.: B25J 7/00, B25J 11/00, B25J 15/00

(54) **ADAPTIVE COMPLIANT ASSEMBLY MECHANISM FOR CROSS-SCALE MICRO-PARTS**

(30) Priority: 27.01.2022 CN 202210103425
(71) Applicant: South China University of Technology, Guangzhou, Guangdong 510640 (CN)
(72) Inventor: ZHANG, Xianmin, Guangzhou, Guangdong 510640 (CN); WANG, Rixin, Guangzhou, Guangdong 510640 (CN); ZHU, Benliang, Guangzhou, Guangdong 510640 (CN)
(74) Representative: Petraz, Gilberto Luigi
(86) International application number: PCT/CN2022/128477
(87) International publication number: WO 2023/142562

(57) **Abstract**

The present invention discloses an adaptive compliant assembly mechanism for cross-scale micro-parts, which includes a compliant gripping mechanism (1), a first piezoelectric stack actuator (2), a second piezoelectric stack actuator (3), and a compliant cellular jaw (4). A first gripping arm (1-1) and a second gripping arm (1-2) are symmetrically arranged about an axis, and both have one end connected to a displacement amplification mechanism (1-3); the first piezoelectric stack actuator (2) and the second piezoelectric stack actuator (3) are respectively embedded in both sides of the displacement amplification mechanism (1-3); and, the compliant cellular jaw (4) is detachably provided at free ends of the first gripping arm (1-1) and of the second gripping arm (1-2). Compared with a conventional assembly mechanism, the adaptive compliant assembly mechanism for cross-scale micro-parts implements adaptive gripping operations of micro-parts with characteristic scales ranging microns to millimeters, has the advantages of compact structure, small volume, and light weight, allows the precise assembly of micro-parts, and can also be used for the compliant assembly of multiple varieties and multiple batches of micro-devices.

## Description

### Technical Field

The invention belongs to the technical field of micro-assembly, and relates particularly to an adaptive compliant assembly mechanism for cross-scale micro-parts.

### Background Art

Micro-assembly is a technology that assembles micro-parts ranging in size from microns to millimeters within a small error range to form a microsystem. It is widely applied in the processing and manufacturing of medical testing equipment, optical communication components, micro-sensors, microsatellite equipment and other products. It is the core and foundation of micro-nano manufacturing field. Assembly mechanisms are components that directly perform operations such as gripping, transporting, and placing micro-assembly objects. They play a crucial role in a micro-assembly system, directly restricting the assembly quality and efficiency of the micro-system, and influencing the design, development and industrial application of a micro-electromechanical system.

Compliant mechanism is the most commonly used form of assembly mechanism, is a type of mechanical structure that relies on its own elastic deformation to transmit force, displacement and energy, and has the advantages of assembly-free, no gap, no friction, and integral fabricating. However, due to the narrow environment of micro-assembly systems and the limited load-bearing capacity of micromanipulators, the sizes and weights of assembly mechanisms are often strictly limited, making it impossible to equip with piezoelectric actuators of large stroke and complex displacement amplification mechanisms, so it is difficult to achieve a large working stroke.

However, with the development of microelectromechanical systems towards the integration of multiple material and functional devices, micro-manipulation tasks require the involvement of micro-parts with different shapes and sizes. These parts characteristic sizes range from microns to millimeters, and the size span far exceeds the maximum working stroke of assembly mechanisms. Therefore, it is difficult for a single assembly mechanism to cope with the assembly requirements of complex microelectromechanical systems. Previous solutions typically involved employing multiple sets of micromanipulators or micro-grippers to achieve adaptive micro-assembly of parts of different shapes and sizes. For example, the Lawrence Livermore National Laboratory in the United States uses up to six sets of manipulating robot systems to assemble inertial confinement fusion micro-targets involving parts of various shapes and sizes. However, this approach significantly increases the difficulty and cost of automated control of assembly equipment. Therefore, there is an urgent need to develop an adaptive assembly mechanism suitable for cross-scale micro-parts. Lofroth and Avci have designed a novel modular compliant gripper, which features mounting holes at its end for installing gripping tips suitable for different objects to be manipulated (Lofroth M, Avci E. Development of a novel modular compliant gripper for manipulation of micro objects [J]. Micromachines, 2019, 10(5): 313.). Nonetheless, the gripper exhibits limitations such as a small displacement amplification ratio, complex configuration, and large structural size. Additionally, the use of bolts for fixing makes it highly susceptible to damage the gripping tips during the installation process, and it is difficult to achieve automated replacement, thereby reducing the efficiency of micro-manipulation tasks.

### Summary of the Invention

### Technical problems

### Solutions to problems

### Technical solutions

The present invention aims to address the limitations of existing assembly mechanisms, which suffer from poor universality and a limited application scope. It provides an adaptive compliant assembly mechanism for cross-scale micro-parts. The present invention can realize adaptive gripping manipulations of micro-parts with characteristic sizes ranging from microns to millimeters. The mechanism has the advantages of compact structure, small volume, light weight. It allows for the precise assembly of micro-parts, and can also be used for the adaptive assembly of multiple varieties and multiple batches of micro-devices.

To achieve the above objectives, the present invention provides an adaptive compliant assembly mechanism for cross-scale micro-parts. The mechanism includes a compliant gripping mechanism, a first piezoelectric stack actuator, a second piezoelectric stack actuator and a compliant cellular jaw.

The compliant gripping mechanism is a symmetric structure that includes a first gripping arm, a second gripping arm, and a displacement amplification mechanism. The first gripping arm and the second gripping arm are arranged symmetrically about an axis, and both have one end connected to the displacement amplification mechanism.

The first piezoelectric stack actuator and the second piezoelectric stack actuator are respectively embedded in both sides of the displacement amplification mechanism.

The compliant cellular jaw is detachably provided at a free end of the first gripping arm and the free end of the second gripping arm. This detachable provision of the compliant cellular jaw facilitates its assembly and replacement. The compliant cellular jaw serves as a replaceable module, enabling the adaptive gripping of micro-parts with various shapes and sizes. When the compliant cell jaw is detached, the compliant gripping mechanism can independently grip micro-parts with particular shapes and sizes.

Preferably, a first notch and a second notch are respectively provided on both sides of the displacement amplification mechanism, wherein the first piezoelectric stack actuator and the second piezoelectric stack actuator are respectively provided in the first notch and the second notch.

Preferably, the compliant gripping mechanism further includes a mounting base, and the root of the displacement amplification mechanism is connected to the mounting base which is provided with mounting holes. Through these mounting holes, micro-grippers can be installed on micromanipulators and precise positioning platforms.

Preferably, the compliant cellular jaw is connected to the first gripping arm and the second gripping arm by snap fit joints.

Preferably, the first gripping surface and the second gripping surface are respectively provided on ends of the first gripping arm and the second gripping arm, which are used not only for positioning and installing the compliant cellular jaw, but also for directly gripping micro-parts. The first gripping surface and the second gripping surface are respectively provided with a first mating part and a second mating part, and the compliant cellular jaw is provided with a first corresponding mating part and a second corresponding mating part. The first corresponding mating part and the second corresponding mating part are respectively matched with the first mating part and the second mating part, to unlock or lock the compliant cellular jaw, facilitating its easy detachment and replacement.

Preferably, the compliant cellular jaw is a symmetrical structure, and includes a first docking mechanism, a second docking mechanism and the flexure beams; the first docking mechanism and the second docking mechanism are arranged symmetrically, and are connected to each other through the flexure beam. Additionally, the compliant cellular jaw is further equipped with gripping surfaces.

Preferably, the gripping surfaces can be provided on the inner side of the first docking mechanism, the inner side of the second docking mechanism, and the outer side of the flexure beam.

Preferably, each gripping surface is designed with embosses which allow for point contact with the parts when gripping the parts. By providing embosses to maintain point contact with micro-parts, surface forces such as adhesion forces, capillary forces between the gripping surfaces and micro-parts is reduced, which not only ensures the gripping stability but also enables the reliable release of micro-parts.

Preferably, when two or more gripping surfaces are provided, the tips of the embosses on all gripping surfaces are located on a circumference of the same inscribed circle whose radius is determined by shapes and sizes of micro-parts. Matching gripping surfaces can be designed for micro-parts of different shapes and sizes.

Specifically, two flexure beams and three gripping surfaces are provided, namely the third gripping surface, the fourth gripping surface and the fifth gripping surface. The third gripping surface is provided on the inner side of the first docking mechanism, the fourth gripping surface is provided on the inner side of the second docking mechanism, and the fifth gripping surface is provided on the outer side of the flexure beam. Embosses are provided at both ends of the third gripping surface, both ends of the fourth gripping surface and at both ends of the fifth gripping surface. The tips of all embosses are located on the circumference of the same inscribed circle. The radius of this inscribed circle can be adjusted to accommodate micro-parts ranging in size from microns to the millimeters.

Preferably, the first docking guide groove and the second docking guide groove are respectively provided at the first docking mechanism and the second docking mechanism. The cross-sections of the first docking guide groove and the second docking guide groove are respectively match with the first gripping surface and the second gripping surface. The first gripping arm and the second gripping arm are detachably connected into the first docking guide groove and the second docking guide groove, respectively.

The overall connection arrangement of the modular compliant micro-gripper for micro-assembly is as follows: the first piezoelectric stack actuator and the second piezoelectric stack actuator are respectively installed in the first notch and the second notch, with interference fit. The pre-tightening of the flexure beam can be achieved by calculating the relationship between the pre-tightening force and the interference amount; the compliant cellular jaw is mounted on both the first gripping arm and the second gripping arm, and is locked cooperatively by the mating parts of the gripping surfaces and the corresponding mating parts of the docking guide grooves.

### Beneficial Effects of the Invention

### Beneficial Effects

Compared with the prior art, the present invention offers several advantageous effects, including but not limited to:
(1) The present invention enables the adaptive gripping of objects ranging in size from microns to millimeters by replacing different modular compliant cellular jaws based on the shapes and sizes of micro-parts, allowing for precise assembly of micro-parts. It facilitates the compliant assembly of multiple varieties and multiple batches of micro-devices.
(2) The embedded piezoelectric stack actuators within the compliant gripping mechanism provides a large displacement amplification ratio and working stroke. With simple and compact structure, small volume and light weight, they can be conveniently installed on micro-manipulation platforms.
(3) The snap fit joints between the compliant gripping mechanism and the compliant cellular ensures a simple structure, fewer components and a stable connection, so that it is easy to achieve automated installation and replacement.
(4) The compliant gripping mechanism is integrally formed by slow-feeding wire cutting, with high stiffness, good integrity, and high movement accuracy.
(5) The compliant cellular jaw uses multiple gripping surfaces and point contact to grip micro-parts, ensuring the gripping stability and the reliable release for micro-parts.

### BRIEF DESCRIPTION OF THE DRAWINGS

### Description of the Drawings

FIG. 1 is an overall structure diagram of an adaptive compliant assembly mechanism for cross-scale micro-parts.
FIG. 2 is a top view of a compliant gripping mechanism.
FIG. 3 is a schematic diagram of the first gripping arm and the second gripping arm of a compliant gripping mechanism.
FIG. 4 is a schematic structure diagram of a compliant cellular jaw.
FIG. 5 is an A-A cross-sectional view of the side view of a compliant cellular jaw.
FIG. 6 is a B-B cross-sectional view of the top view of a compliant cellular jaw.
FIG. 7 is a schematic diagram of the buckle connection between a compliant cellular jaw and a compliant gripping mechanism.

In the drawings: 1. compliant gripping mechanism; 1-1: first gripping arm; 1-1-1: first gripping surface; 1-1-2: first mating part; 1-2: second gripping arm; 1-2-1: second gripping surface; 1-2-2: second mating part; 1-3: displacement amplification mechanism; 1-3-1: first notch; 1-3-2: second notch; 1-4: mounting base; 2: first piezoelectric stack actuator; 3: second piezoelectric stack actuator; 4: compliant cellular jaw; 4-1: third gripping surface; 4-2: fourth gripping surface; 4-3: fifth gripping surface; 4-4: first flexure beam; 4-5: second flexure beam; 4-6: first docking mechanism; 4-6-1: first docking guide groove; 4-6-2: first corresponding mating part; 4-7: second docking mechanism; 4-7-1: second docking guide groove; and 4-7-2: second corresponding mating part.

### DETAILED DESCRIPTION OF THE INVENTION

Specific embodiments of the present invention will be further described in detail below in combination with the accompanying drawings. It should be noted that these specific embodiments are only specific illustrations and are not the limitation to the present invention.

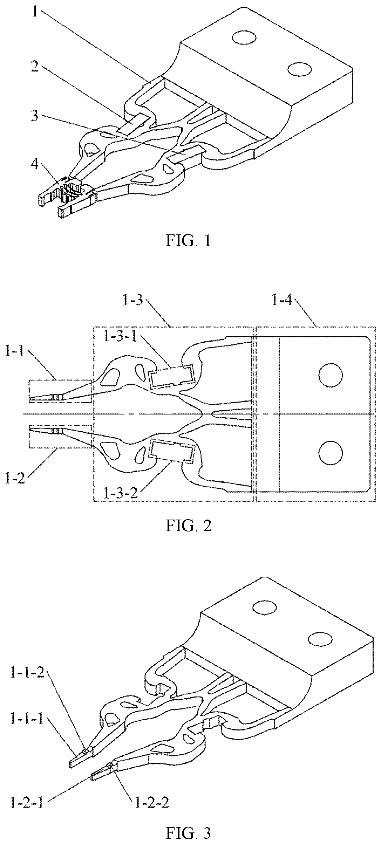

Referring to FIG. 1, an adaptive compliant assembly mechanism for cross-scale micro-parts provided in the present invention, has a replaceable compliant cell jaw 4 and is suitable for adaptive assembly of micro-parts with characteristic scales ranging from microns to millimeters. This mechanism enables batch assembly of micro-parts, and can also be used for compliant assembly of small batches, multiple varieties, and multiple batches of micro-devices.

Referring to FIG. 1, the adaptive compliant assembly mechanism for cross-scale micro-parts provided in the present invention includes a compliant gripping mechanism 1, a first piezoelectric stack actuator 2, a second piezoelectric stack actuator 3 and a compliant cellular jaw 4.

In the present invention, referring to FIG. 2, the compliant gripping mechanism 1 is a symmetrical structure processed using slow-feeding wire cutting, which includes a first gripping arm 1-1, a second gripping arm 1-2, a displacement amplification mechanism 1-3 and a mounting base 1-4. The first gripping arm 1-1 and the second gripping arm 1-2 are arranged symmetrically about an axis, and connected to the displacement amplification mechanism 1-3 whose root is connected to the mounting base 1-4. The displacement amplification mechanism 1-3 features a first notch 1-3-1 and a second notch 1-3-2 on both sides. The cross-sectional shapes of the first notch 1-3-1 and the second notch 1-3-2 are adapted to the shapes of the piezoelectric stack actuators. The first piezoelectric stack actuator 2 and the second piezoelectric stack actuator 3 are respectively provided within the first notch 1-3-1 and the second notch 1-3-2. The displacement amplification mechanism 1-3 is used to amplify the output displacement of the piezoelectric actuators. The mounting base 1-4 is equipped with two mounting holes for installing the compliant assembly mechanism provided in the present invention onto other platforms.

In some embodiments of the present invention, the compliant gripping mechanism 1 is a planar symmetrical structure. In other embodiments, the gripping mechanism may be configured as a three-dimensional axisymmetric structure to have multiple gripping arms.

In some embodiments of the present invention, the mounting holes are circular through holes.

In some embodiments of the present invention, the first notch 1-3-1 and the second notch 1-3-2 are located on both sides of the middle part of the displacement amplification mechanism 1-3. The cross-sectional shapes of the piezoelectric stack actuator, the first notch 1-3-1 and the second notch 1-3-2 are rectangular. Of course, in other embodiments, if the piezoelectric actuators have other shapes, the first notch 1-3-1 and the second notch 1-3-2 are also adjusted according to the shapes of the piezoelectric actuators.

In the present invention, referring to FIG. 3, the first gripping surface 1-1-1 and the second gripping surface 1-2-1 are respectively provided on the ends of the the first gripping arm 1-1 and the second gripping arm 1-2; the upper surfaces of the roots of the first gripping surface 1-1-1 and the second gripping surface 1-2-1 are respectively provided with a first mating part 1-1-2 and a second mating part 1-2-2 with embosses which can improve the clamping effect. In other embodiments, the snap fit joints can also be provided in other forms or at other locations according to actual needs.

In some embodiments of the present invention, the first gripping surface 1-1-1 and the second gripping surface 1-2-1 have a wedged cross-sectional shape.

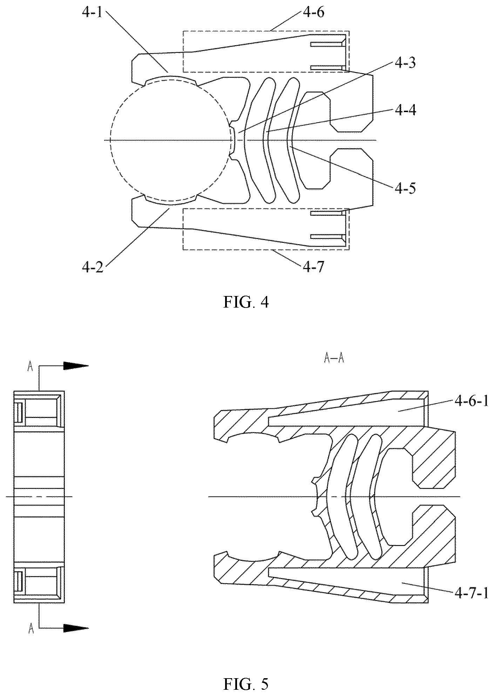

In some embodiments of the present invention, referring to FIG. 4, the compliant cellular jaw 4 is a symmetrical structure, including a third gripping surface 4-1, a fourth gripping surface 4-2, a fifth gripping surface 4-3, a first flexure beam 4-4, a second flexure beam 4-5, a first docking mechanism 4-6 and a second docking mechanism 4-7. The first docking mechanism 4-6 and the second docking mechanism 4-7 are arranged symmetrically about an axis, and are connected by the fifth gripping surface 4-3, the first flexure beam 4-4 and the second flexure beam 4-5. The third gripping surface 4-1 and the fourth gripping surface 4-2 are respectively connected to the first docking mechanism 4-6 and the second docking mechanism 4-7. Both ends of the third gripping surface 4-1, both ends of the fourth gripping surface 4-2 and both ends of the fifth gripping surface 4-3 are provided with embosses to maintain point contact with micro-parts, thus ensuring the gripping stability and the reliable release for micro-parts. The tips of the embosses are all located on the circumference of the same inscribed circle whose radius is determined by micro-parts. Matching gripping surfaces may be designed for micro-parts of different shapes and sizes.

Wherein, the flexure beams have the function of connecting the two docking mechanisms with each other and increasing the stiffness of the entire structure. In other embodiments, one flexure beam or other number of flexure beams may be provided.

Wherein, the number of gripping surfaces in the compliant cellular jaw 4 can also be set as other values.

In some embodiments of the present invention, referring to FIG. 5, the first docking guide groove 4-6-1 and the second docking guide groove 4-7-1 are respectively provided in the first docking mechanism 4-6 and the second docking mechanism 4-7.

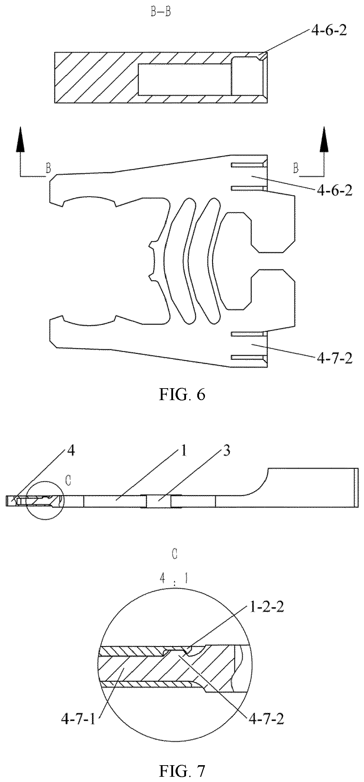

In some embodiments of the present invention, referring to FIG. 6, the roots of the first docking guide groove 4-6-1 and the second docking guide groove 4-7-1 are respectively equipped with a first corresponding mating part 4-6-2 and a second corresponding mating part 4-7-2 with embosses, which are used to cooperate with the first mating part 1-1-2 and the second mating part 1-2-2 to complete the locking of the compliant cellular jaw 4. Wherein, the docking guide grooves can guide the compliant cellular jaw to a mounting position, thereby reducing the requirements for the absolute positioning accuracy of micro-manipulating mechanisms and making it easier to realize automated installation and replacement. The provision of embosses can enhance the clamping effect.

An adaptive compliant assembly mechanism for cross-scale micro-parts provided in the embodiments of the present invention has the overall connection relationship as follows: the first piezoelectric stack actuator 2 and the second piezoelectric stack actuator 3 are respectively installed with an interference fit in the first notch 1-3-1 and the second notch 1-3-2 to achieve the pre-tightening of the piezoelectric stack actuators; the compliant cellular jaw 4 is installed on the first gripping arm 1-1 and the second gripping arm 1-2, and is fixed by snap fit joints; referring to FIG. 7, the specific fixing mode is as follows: the first gripping surface 1-1-1 is inserted into the first docking guide groove 4-6-1 and is locked through the first corresponding mating part 4-6-2 and the first mating part 1-1-2, while the second gripping surface 1-2-1 is inserted into the second docking guide groove 4-7-1 and is locked through the second corresponding mating part 4-7-2 and the second mating part 1-2-2. Wherein, the piezoelectric stack actuators are embedded inside the compliant gripping mechanism while optimizing the positions of the actuators and the configuration of the compliant mechanism, thus obtaining the compliant mechanism with a larger working stroke. Based on the lever principle, the embedding of the piezoelectric stack actuators inside the compliant gripping mechanism, the ratio of effort arm to load arm can be made larger, thereby achieving a larger displacement amplification ratio and working stroke.

The compliant cellular jaw 4 is a replaceable module. The tips of the embosses on the third gripping surface 4-1, fourth gripping surface 4-2 and fifth gripping surface 4-3 are located on the circumference of the same inscribed circle whose radius is adapted to the shapes and sizes of micro-parts, enabling adaptive gripping of micro-parts of various shapes and sizes. The compliant gripping mechanism 1 can also work independently, with the first gripping arm 1-1 and the second gripping arm 1-2 capable of gripping micro-parts.

The working process of the compliant assembly mechanism provided in the embodiments of the present invention is as follows: firstly, the compliant assembly mechanism is assembled as shown in the figures, and is installed on a micro-manipulation platform; when gripping micro-parts, the first piezoelectric stack actuator 2 and the second piezoelectric stack actuator 3 are respectively loaded with driving voltages, and the output force of the actuators causes the displacement amplification mechanism 1-3 to deform, driving the first gripping arm 1-1 and the second gripping arm 1-2 move toward each other; this enables the gripping of micro-parts of fixed sizes. When the compliant cellular jaw 4 is provided, the compliant cell jaw 4 can be further driven to work. When the compliant gripping mechanism 1 is equipped with the compliant cellular jaw 4, the first gripping arm 1-1 and the second gripping arm 1-2 drive the third gripping surface 4-1, fourth gripping surface 4-2 and the fifth gripping surface 4-3 to move; wherein, the third gripping surface 4-1 and the fourth gripping surface 4-2 move perpendicular to the axis toward each other, while the movement direction of the fifth gripping surface 4-3 is parallel to the axis, thereby realizing a three-finger gripping of micro-parts. When there is a need for griping micro-parts of different sizes and shapes, the original compliant cell jaw 4 can be disassembled and is replaced with a matching compliant cell jaw 4, thus achieving the gripping of micro-parts of different sizes and shapes. When releasing micro-parts, the power supplies of the first piezoelectric stack actuator 2 and the second piezoelectric stack actuator 3 are turned off, and the compliant gripping mechanism 1 returns to its original state, with the first gripping arm 1-1 and the second gripping arm 1-2 open, driving the third gripping surface 4-1, the fourth gripping surface 4-2 and the fifth gripping surface 4-3 to open, so that micro-parts are released.

The above embodiments of the present invention are only for clearly illustrating the examples provided in the present invention, and are not intended to limit the implementations of the present invention. For those skilled in the art, other different forms of changes or modifications can be made based on the above description. An exhaustive list of all implementations is neither necessary nor possible here. Any modifications, equivalent substitutions and improvements made within the spirit and principles of the present invention should be included in the protection scope of the claims of the present invention.

## Claims

1. An adaptive compliant assembly mechanism for cross-scale micro-parts, **characterized by** comprising a compliant gripping mechanism (1), a first piezoelectric stack actuator (2), a second piezoelectric stack actuator (3) and a compliant cellular jaw (4);
wherein the compliant gripping mechanism (1) is a symmetrical structure and comprises a first gripping arm (1-1), a second gripping arm (1-2), and a displacement amplification mechanism (1-3); the first gripping arm (1-1) and the second gripping arm (1-2) are arranged symmetrically about an axis and both have one end connected to the displacement amplification mechanism (1-3);
the first piezoelectric stack actuator (2) and the second piezoelectric stack actuator (3) are respectively embedded in both sides of the displacement amplification mechanism (1-3); and
the compliant cellular jaw (4) is detachably provided at a free end of the first gripping arm (1-1) and a free end of the second gripping arm (1-2).

2. The adaptive compliant assembly mechanism for cross-scale micro-parts according to claim 1, **characterized in that**, a first notch (1-3-1) and a second notch (1-3-2) are respectively provided on both sides of the displacement amplification mechanism (1-3), and the first piezoelectric stack actuator (2) and the second piezoelectric stack actuator (3) are respectively provided in the first notch (1-3-1) and the second notch (1-3-2).

3. The adaptive compliant assembly mechanism for cross-scale micro-parts according to claim 1, **characterized in that**, the compliant gripping mechanism (1) is capable of being configured as a three-dimensional axisymmetric structure, and comprises multiple gripping arms.

4. The adaptive compliant assembly mechanism for cross-scale micro-parts according to claim 1, **characterized in that**, the compliant cellular jaw (4) is connected to the first gripping arm (1-1) and the second gripping arm (1-2) by buckle connection.

5. The adaptive compliant assembly mechanism for cross-scale micro-parts according to claim 4, **characterized in that**, a first gripping surface (1-1-1) and a second gripping surface (1-2-1) on are respectively provided on ends of the first gripping arm (1-1) and the second gripping arm (1-2), the first gripping surface (1-1-1) and the second gripping surface (1-2-1) are respectively provided with a first mating part (1-1-2) and a second mating part (1-2-2), and the compliant cellular jaw (4) is provided with a first corresponding mating part (4-6-2) and a second corresponding mating part (4-7-2) that are respectively matched with the first mating part (1-1-2) and the second mating part (1-2-2), to unlock or lock the compliant cellular jaw (4).

6. The adaptive compliant assembly mechanism for cross-scale micro-parts according to any one of claims 1-5, **characterized in that**, the compliant cellular jaw (4) is a symmetrical structure and comprises a first docking mechanism (4-6), a second docking mechanism (4-7) and a flexure beam (4-5); the first docking mechanism (4-6) and the second docking mechanism (4-7) are arranged opposite to each other, and are connected to each other through the flexure beam (4-5); and, the compliant cellular jaw (4) is further provided with gripping surfaces.

7. The adaptive compliant assembly mechanism for cross-scale micro-parts according to claim 6, **characterized in that**, the gripping surfaces are capable of being provided on an inner side of the first docking mechanism (4-6), an inner side of the second docking mechanism (4-7), and an outer side of the flexure beam (4-5).

8. The adaptive compliant assembly mechanism for cross-scale micro-parts according to claim 7, **characterized in that**, each gripping surface is provided with embosses, and, when gripping parts, each emboss is in point contact with the parts.

9. The adaptive compliant assembly mechanism for cross-scale micro-parts according to claim 7, **characterized in that**, when two or more gripping surfaces are provided, tips of the embosses on all gripping surfaces are located on the circumference of a same inscribed circle whose radius is determined by shapes and sizes of micro-parts.

10. The adaptive compliant assembly mechanism for cross-scale micro-parts according to claim 6, **characterized in that**, a first docking guide groove (4-6-1) and a second docking guide groove (4-7-1) are respectively provided at the first docking mechanism (4-6) and the second docking mechanism (4-7), and the first gripping arm (1-1) and the second gripping arm (1-2) are detachably connected into the first docking guide groove (4-6-1) and the second docking guide groove (4-7-1) respectively.
